# EUROPEAN PATENT APPLICATION

(11) **EP 3 305 200 A1**
(43) Date of publication of application: **11.04.2018**
(21) Application number: 16192796.7
(22) Date of filing: 07.10.2016
(51) Int. Cl.: A61B 6/04, A61B 6/00, A61B 8/00, A61N 5/10

(54) **MEDICAL APPARATUS COMPRISING A HADRON THERAPY DEVICE, A MRI, AND A PROMPT-GAMMA SYSTEM**

(71) Applicant: ION BEAM APPLICATIONS S.A., 1348 Louvain-la-Neuve (BE)
(72) Inventor: PRIEELS, Damien, 1348 Louvain-la-Neuve (BE)
(74) Representative: Pecher, Nicolas

(57) **Abstract**

Medical apparatus that comprises:
(A) a hadron therapy device adapted for directing a hadron beam having an initial beam energy, **E0**, along a beam path to a target spot located inside a subject of interest;
(B) a MRI for acquiring magnetic resonance (MR) image within an imaging volume, Vp, comprising the target spot;
(C) a prompt-γ system adapted for acquiring a signal generated by the hadron beam;
(D) a controller configured for:
• computing an actual position of the Bragg peak of said hadron beam, based on the signal acquired by the PG system, and
• locating the actual position of the Bragg peak on an MR image of the imaging volume, Vp, acquired with the MRI along the beam path from an outer surface of the subject of interest to the target spot.

## Description

### Field of the invention

According to a first aspect, the invention relates to a medical apparatus comprising a charged hadron therapy device, a magnetic resonance imaging device, and a prompt-γ system. According to a second aspect, the invention relates to method for checking a treatment plan.

### Description of prior art

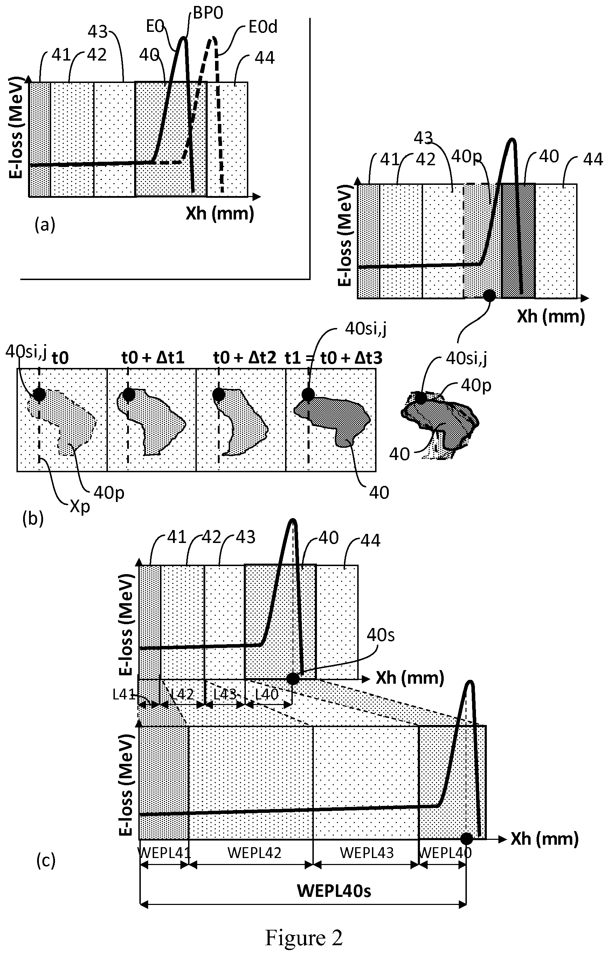

Hadron therapy (for example, proton therapy) for treating a patient has been known for a couple of decades with the prospect of several advantages over conventional radiotherapy. These advantages are due to the physical nature of hadrons. A photon beam in conventional radiotherapy releases its energy according to a decreasing exponential curve as a function of the distance of tissue traversed by the photon beam. By contrast and such as illustrated in Figure 2, a hadron beam first releases a small fraction of its energy as it penetrates tissues **41-43,** forming a plateau then, as the hadron path is prolonged, the whole energy is released locally following a steep increase to a peak and a fall-off at the end of the range of the beam. The peak is called Bragg peak and corresponds to the maximum of the Bragg curve illustrated in Figure 2(c). Consequently, a hadron beam can deliver a high dose of hadrons at a precise location within a target tissue **40,** thus preserving the surrounding healthy tissues **41-44.** As illustrated in Figure 2(a), the advantage of hadron therapy allowing the delivery of high doses of hadron at a precise location is also one of its weaknesses, because if the position, **BP0,** of the Bragg peak of a hadron beam is offset relative to the target tissues **40,** high doses of hadrons may be delivered to adjacent tissues **43, 44** which are healthy (compare solid line, **E0**, and dashed line, **E0d,** of the curves of energy loss, Eₗₒₛₛ, with respect to the distance, **Xh,** travelled by the hadron beam within tissues and measured along the beam path, **Xp,** in Figure 2(a)). For this reason, the determination of the relative position of the Bragg peak with respect to the position of the target tissue is crucial to properly implement hadron therapy to a patient.

In practice, hadron therapy usually requires the establishment of a treatment plan before any treatment can start. During this treatment plan, a computer tomography scan (CT scan) of the patient and target tissues is usually performed. The CT scan is used to characterize the target tissue **40** and the surrounding tissues **41-43** to be traversed by a treatment hadron beam **1h** for the treatment of a patient. The characterization yields a 3D representation of the volume comprising the target tissue, and a treatment plan system determines a range-dose calculated based on the nature of the tissues **41-43** traversed by the hadron beam.

This characterization permits computation of a water equivalent path length (WEPL), which is used for determining the initial energy, **Ek,** of the treatment hadron beam required for delivering a prescribed dose of hadrons to a target spot **40s,** wherein k = 0 or 1 depending on the stage when said initial energy was determined. Figure 2(c) illustrates the conversion of the physical distances travelled by a hadron beam traversing different tissues into corresponding WEPL's. The WEPL of a hadron beam travelling a given distance through a given tissue is the equivalent distance said hadron beam would travel in water. As illustrated in Figure 2(c) if, as is usually the case, healthy tissues **41-43** of different natures and thicknesses separate a target tissue from the outer surface of the skin of a patient, the WEPL of a target spot is calculated taking into account the water corresponding path lengths of each tissue in series until the target spot is reached. With a value of the equivalent path length of a hadron beam traveling in water, the initial energy, **Ek,** required for positioning the Bragg peak at the WEPL of the target spot can easily be computed and corresponds to the initial energy, **Ek,** required for positioning the Bragg peak at the target spot within the target tissue.

The treatment plan can then be executed during a treatment phase including one or more treatment sessions during which doses of hadrons are deposited onto the target tissue. The position of the Bragg peak of a hadron beam with respect to the target spots of a target tissue, however, suffers of a number of uncertainties including:
- the variations of the patient position, on the one hand, during a hadron therapy session and, on the other hand, between the establishment of the treatment plan and the hadron therapy session;
- the variations of the size and/or of the position of the target tissue (see Figure 2(b)) and/or of the healthy tissues **41-43** positioned upstream from the target tissue with respect to the hadron beam.
- the range calculation from CT scans is limited by the quality of the CT images. Another limitation is linked to the fact that CT scans use the attenuation of X-rays that have to be converted in hadron attenuation which is non obvious and depends on the chemical composition of the tissues traversed.

The uncertainty on the position of the patient and, in particular, of the target tissue is critical for obvious reasons. Even with an accurate characterization by CT scan, the actual position of a target tissue during a treatment session remains difficult to ascertain for the following reasons:
- first, during an irradiation session, the position of a target tissue can change because of anatomical processes such as breathing, digestion, or heartbeats of the patient. Anatomical processes can also cause gases or fluids appearing or disappearing from the beam path, **Xp,** of a hadron beam.
- second, treatment plans are usually determined several days or weeks before a hadron treatment session starts and treatment of a patient can take several weeks distributed over several treatment sessions. During this time period, the patient can lose or gain weight, therefore modifying, sometimes significantly, the volume of tissues such as fats and muscles.

Accordingly, the size of the target tissue can change (e.g. a tumour may have grown, receded, or changed position or geometry). Figure 2(b) shows an example of evolution of the size and position of a target tissue **40** between the time, **t0,** of the establishment of the treatment plan and the times, **t0** + **Δt1, t0** + **Δt2, t1** = **t0** + **Δt3,** of treatment sessions. The treatment plan and last treatment session may be separated by several days or weeks. The treatment plan established at time, **t0,** may therefore comprise irradiation of a target spot **40si,j** (black spot in Figure 2(b)) which belonged to the target tissue **40p** at said time, **t0.** Because the target tissue **40p** may have moved or changed shape during the period, **Δt3,** said target spot **40si,j** may not belong to the target tissue **40** anymore at the time, **t0** + **Δt3,** of the treatment session and be located in a healthy tissue instead. Consequently, irradiating said target spot would hit and possibly harm healthy tissues **43** instead of target tissues **40.**

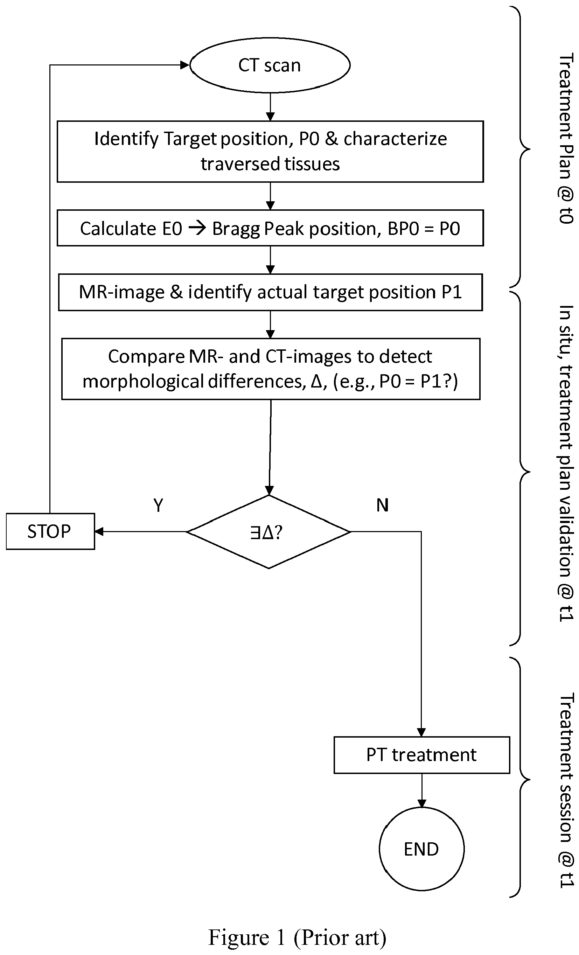

The use of a magnetic resonance imaging device (MRI) coupled to a hadron therapy device has been proposed in the art for identifying any variation of the size and /or the position of a target tissue. For example, US patent 8427148 describes a system comprising a hadron therapy device coupled to a MRI. Said system can acquire images of the patient during a hadron therapy session and can compare these images with CT scan images of the treatment plan. Present Figure 1 illustrates an example of a known flowchart of a hadron therapy session using a hadron therapy device coupled to an MRI. A treatment plan is established including the characterization of the target tissue **40s** and surrounding tissues **41-43.** This step is traditionally performed with a CT scan analysis and allows the determination of the position, **P0**, and morphology of a target tissue, the best trajectories or beam paths, **Xp,** of hadron beams for the hadron treatment of the target tissue, and characterization of the sizes and natures of the tissues traversed by a hadron beam following said beam paths, **Xp,** to determine WEPL40s's of target spots of the said target tissue. The initial energies, **Ek,** of the hadron beams required for matching the corresponding positions, **BP0,** of the Bragg peaks of the hadron beams to the position, **P0**, of the target tissue can thus be calculated. This completes the establishment of a treatment plan.

A hadron therapy session follows the establishment of the treatment plan. With an MRI coupled to a hadron therapy device, it is possible to capture a magnetic resonance (MR) image of a volume, **Vp,** including the target tissue and surrounding tissues to be traversed by a hadron beam. The MR image can then be compared with CT scan images to assess whether any morphological differences, Δ, can be detected in the imaged tissues between the time the CT scans were performed (= **t0** in Figure 2(b)) and the time of the hadron therapy session (**t1**= **t0** + **Δ t3** in Figure 2(b)). If no substantial difference in morphology affecting the treatment session can be detected, then the hadron therapy session proceeds as planned in the treatment plan. If, on the other hand, some differences are detected that could influence the relative position of the target tissue with respect to the planned hadron beams and their respective Bragg peaks, the hadron therapy session is interrupted and a new treatment plan must be established. This technique proposed in the art is greatly advantageous because it can prevent carrying out a hadron therapy session based on a treatment plan which has become obsolete, thus preventing healthy tissues from being irradiated instead of the target tissue.

The magnetic resonance (MR) images provide high contrast of soft tissue traversed by a hadron beam but, to date, have not been suitable for visualizing the hadron beam itself, let alone the position of the Bragg peak because:
- MRI measures the density of hydrogen atoms in tissues, but to date does not yield any identifiable information on the hadron stopping power ratio. The conversion from density of hydrogen atoms to the hadron stopping power ratio suffers from uncertainties similar to and yet less understood than those of the conversion from X-rays in CT scan.
- Due to the different techniques used in CT scan and in MRI, the comparison between the images from CT scan and the images from MRI is not obvious and can suffer from uncertainties.

In conclusion, whilst in hadron therapy, an accurate determination of the position of the Bragg peak relative to the portion of a target tissue is crucial because errors on this position may lead to the irradiation of healthy tissues rather than irradiation of target tissues, no satisfactory solution for determining the relative positions of the Bragg peak and target tissues is available to date. Apparatuses combining a hadron therapy device and a MRI proposed in the art allow *in situ* acquisition of images during a treatment session thus giving information related to the actual position of the target tissue. Said images are, however, not sufficient for ensuring the exact determination of the position of the Bragg peak of a hadron beam and of where it stands relative to the target tissue. There therefore remains a need for a hadron therapy device combined to MRI allowing a better determination of the position of the Bragg peak relative to the position of a target tissue.

### Summary of the invention

The present invention is defined in the appended independent claims. Preferred embodiments are defined in the dependent claims.

According to a first aspect, the invention relates to a medical apparatus comprising:
(A) a hadron therapy device comprising a hadron source adapted for directing a hadron beam having an initial beam energy, E0, along a beam path to a target spot located inside a subject of interest;
(B) a magnetic resonance imaging device (MRI) for acquiring magnetic resonance (MR) image within an imaging volume, Vp, comprising the target spot;
(C) a prompt-γ system adapted for acquiring a signal generated by the hadron beam;
(D) a controller configured for:
   - computing an actual position, BP1, of the Bragg peak of said hadron beam, based on the signal acquired by the prompt-γ system; and
   - locating the actual position, BP1, of the Bragg peak on an MR image of the imaging volume, Vp, acquired with the MRI along the beam path from an outer surface of the subject of interest to the target spot.

The medical apparatus can further comprise a display, and the controller can be configured for representing on a same coordinate scale the MR image obtained from the MRI and the position of the Bragg peak obtained from the prompt-γ system.

The controller can also be configured for comparing the actual position, BP1, of the Bragg peak and the actual position, P1, of the target spot.

Preferably, wherein in case the actual position, BP1, and the position, P1, of the target spot are offset by a distance greater than a given tolerance, the controller is further configured for computing the water equivalent path lengths of each tissue m, crossed by the beam path and comprised between the outer surface and the target spot. The computation may be based on the thickness Lm and nature of each tissue m determined on the MR image, and on the water equivalent path length corresponding to the distance between the outer surface and the target spot determined by the prompt-γ system.

The tolerance is preferably lower than ±10 mm, preferably ± 5 mm, more preferably ± 3 mm.

Preferably, the controller controller is configured for optimising the treatment plan by correcting the value of planned initial beam energy E0 of target spot, to a corrected initial beam energy E1, suitable for matching the positions of the Bragg peak of said hadron beam with the positions of all target spots located in a same iso-energy volume, Vti.

In an alternative embodiment, the prompt-γ system is replaced by at least one of a PET system and an ultrasound system.

The medical apparatus according to the present invention can further comprise a a hadron radiography system and/or a support for supporting a patient in a non-supine position.

According to a second aspect, the invention relates to a method for locating the Bragg peak of a hadron beam having an initial beam energy, E0 and being emitted along a beam path to a target spot within a target tissue. The method comprises the following steps:
(A)performing a magnetic resonance (MR) imaging of an imaging volume, Vp, comprising a target spot, and acquiring MR image;
(B) emitting, along the beam path to the target spot, the hadron beam having an initial beam energy, E0;
(C) detecting a signal generated by said hadron beam with a prompt-γ system;
(D)from said signal, determining an actual position, BP1, of the Bragg peak of said hadron beam, based on the signal acquired by the prompt-γ system;
(E) locating the actual position, BP1, of the Bragg peak on an MR image of the imaging volume, Vp, acquired with the MRI along the beam path from an outer surface of the subject of interest to the target spot.

The method can further comprise comprising the step of comparing the actual position, BP1, of the Bragg peak and the actual position, P1, of the target spot.

Preferably, , wherein in case the actual position, BP1, and the actual position, P1, of the target spot are offset by a distance greater than a given tolerance, the method further comprises the step of computing the water equivalent path lengths of each tissue m, crossed by the beam path and comprised between the outer surface and the target spot. The computation may be based on the thickness Lm and nature of each tissue m determined on the MR image, and on the water equivalent path length corresponding to the distance between the outer surface and the target spot determined by the prompt-γ system.

The tolerance is preferably lower than ±10 mm, preferably ± 5 mm, more preferably ± 3 mm.

Preferably, the method comprises the the step of optimising the treatment plan by correcting the value of planned initial beam energy E0 of target spot, to a corrected initial beam energy E1, suitable for matching the positions of the Bragg peak of said hadron beam with the positions of all target spots located in a same iso-energy volume, Vti.

The method can further comprises the steps of:
(F) providing a display;
(G) representing on a same coordinate scale the magnetic resonance data obtained from the MRI and the actual position of the Bragg peak obtained from the prompt-γ system.

Preferably, the steps of performing a magnetic resonance (MR) imaging and of emitting an imaging hadron beam are done in the same room.

Preferably, the method further comprises the step of a medical apparatus according to the present invention.

### Short description of the drawings

These and further aspects of the invention will be explained in greater detail by way of example and with reference to the accompanying drawings in which:
**Figure 1** shows a flowchart of a state of the art hadron therapy method using a hadron therapy device coupled to a MRI;
**Figure 2** schematically shows (a) the position of the Bragg peak of a hadron beam traversing tissues, (b) that changes with time of the morphology and position of a target tissue can create a discrepancy between a treatment plan and an actual required treatment, and (c) relationship between actual path lengths and water equivalent path lengths.
**Figure 3** schematically shows two embodiments of a medical apparatus comprising a hadron therapy device coupled to a MRI;
**Figure 4** schematically illustrates the hadron pencil beam treatment of a target tissue;
**Figure 5** schematically shows a selection of an imaging slice, Vpi, in a MRI and creation of phase gradients and frequency gradients;
**Figure 6** shows two examples of an apparatus according to the present invention, showing access of a hadron beam to a target tissue;
**Figure 7** shows an example of a medical apparatus comprising a hadron therapy device, a MRI device, and a prompt-γ system according to the present invention;
**Figure 8** schematically illustrates an example of detector for prompt-γ system;
**Figure 9** schematically illustrates the computation of the energy of a hadron beam according to the present invention;
**Figure 10** show flowchart of a hadron therapy method using a medical apparatus according to the present invention;
**Figure 11** shows an example of a medical apparatus comprising a hadron therapy device, a MRI device, and a PET scan according to the present invention;
**Figure 12** shows an example of a medical apparatus comprising a hadron therapy device, a MRI device, and an ultrasound system according to the present invention;
**Figure 13** shows an embodiment of a medical apparatus according to the present invention and further comprising a hadron radiography system;
**Figure 14** shows an embodiment of a medical apparatus according to the present invention and further comprising a support for supporting a patient in a non supine position.

The figures are not drawn to scale. Generally, identical components are denoted by the same reference numerals in the figures.

### Detailed description of preferred embodiments

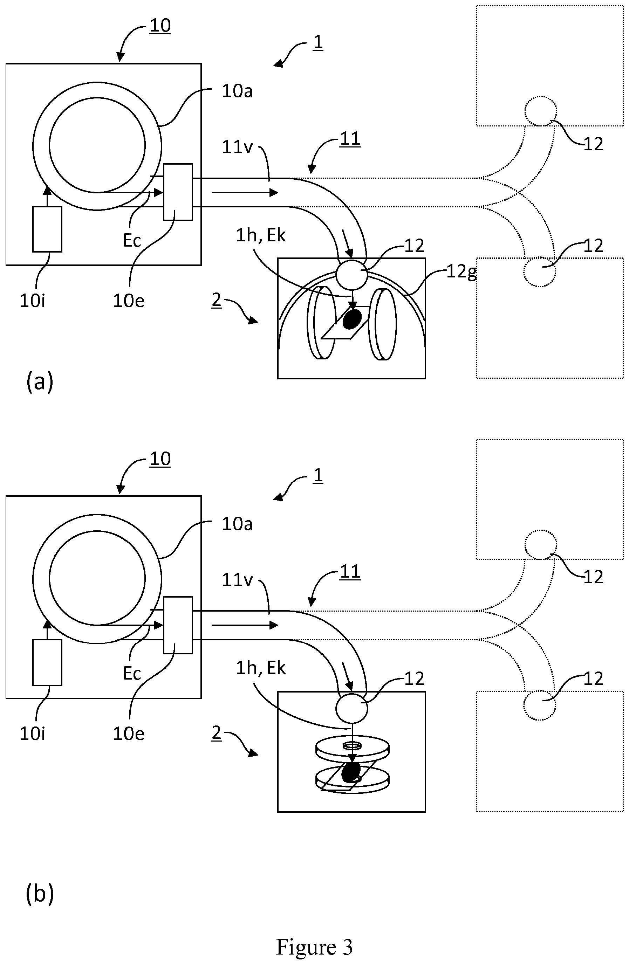

Hadron therapy is a form of external beam radiotherapy using beams **1h** of energetic hadrons. Figures 3, 4&6 show a hadron beam **1h** directed towards a target spot **40s** in a target tissue **40** of a subject of interest. Target tissues **40** of a subject of interest typically include cancerous cells forming a tumour. During a hadron therapy session, a hadron beam of initial energy, **Ek,** with k = 0 or 1, irradiates one or more target spots within the target tissue, such as a tumour, and destroys the cancerous cells included in the irradiated target spots, thus reducing the size of the treated tumour by necrosis of the irradiated tissues.

The subject of interest may comprise a plurality of materials including organic materials. Preferably, the subject of interest comprises a plurality of tissues **m,** with m = 40-44 as shown in Figure 2, that can be, for example: skin, fat, muscle, bone, air, water (blood), organ, and tumour. The target tissue **40** is preferably a tumour.

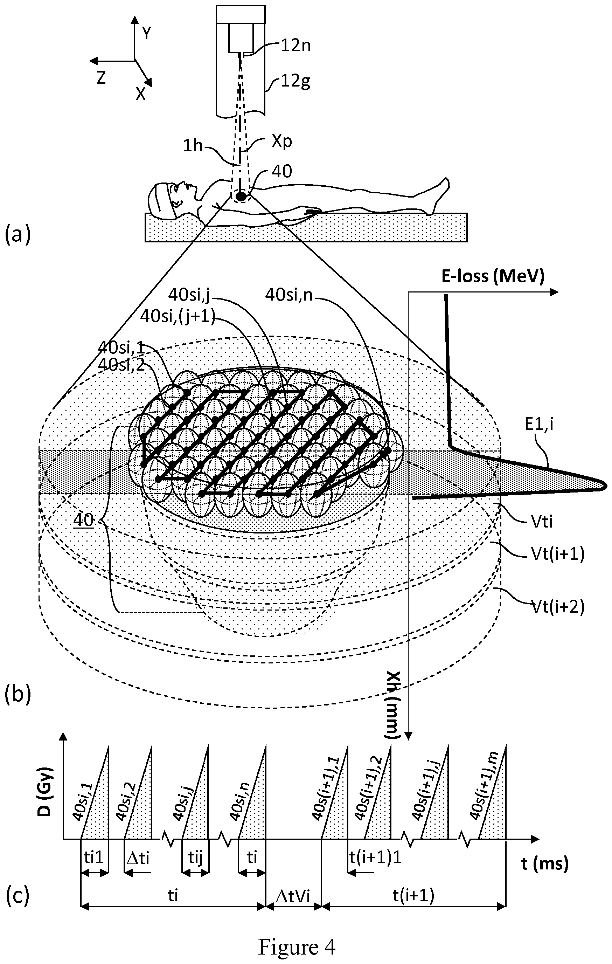

As described in the prior art literature, a hadron beam **1h** traversing an organic body along a beam path, **Xp,** loses most of its energy at a specific distance of penetration along the beam path, **Xp.** As illustrated in Figures 2&4, said specific distance of penetration corresponds to the position of the so called Bragg peak, observed when plotting the energy loss per unit distance [MeVg⁻¹cm⁻²], **Eₗₒₛₛ,** of a hadron beam as a function of the distance, **xh,** measured along the beam path, **Xp.** Unlike other forms of radiation therapies, a hadron beam can thus deliver a high dose of energy at a very specific location within a target tissue corresponding to the position of the Bragg peak. The position of the Bragg peak depends mainly on the initial energy, **Ek,** of the hadron beam (i.e., before traversing any tissue) and on the nature and thicknesses of the traversed tissues. The hadron dose delivered to a target spot depends on the intensity of the hadron beam and on the time of exposure. The hadron dose is measured in Grays (Gy), and the dose delivered during a treatment session is usually of the order of one to several Grays (Gy).

A hadron is a composite particle made of quarks held together by strong nuclear forces. Typical examples of hadrons include protons, neutrons, pions, and heavy ions, such as carbon ions. In hadron therapy, electrically charged hadrons are generally used. Preferably, the hadron is a proton and the corresponding hadron therapy is referred to as proton therapy. In the following, unless otherwise indicated, any reference to a proton beam or proton therapy applies to a hadron beam or hadron therapy in general.

A hadron therapy device **1** generally comprises a hadron source **10,** a beam transport line **11,** and a beam delivery system **12.** Charged hadrons may be generated from an injection system **10i,** and may be accelerated in a particle accelerator **10a** to build up energy. Suitable accelerators include for example, a cyclotron, a synchro-cyclotron, a synchrotron, or a laser accelerator. For example, a (synchro-)cyclotron can accelerate charged hadron particles from a central area of the (synchro-)cyclotron along an outward spiral path until they reach the desired output energy, **Ec,** whence they are extracted from the (synchro-)cyclotron. Said output energy, **Ec,** reached by a hadron beam when extracted from the (synchro-)cyclotron is typically comprised between 60 and 400 MeV, preferably between 210 and 250 MeV. The output energy, **Ec,** is not necessarily the initial energy, **Ek,** of the hadron beam used during a therapy session; **Ek** is equal to or lower than **Ec, Ek** ≤ **Ec.** An example of a suitable hadron therapy device includes, but is not limited to, a device described in US Pat. No. 4870287, the entire disclosure of which is incorporated herein by reference as representative of a hadron beam therapy device as used in the present invention.

The energy of a hadron beam extracted from a (synchro-)cyclotron can be decreased by energy selection means **10e** such as energy degraders, positioned along the beam path, **Xp,** downstream of the (synchro-)cyclotron, which can decrease the output energy, **Ec,** down to any value of **Ek** including down to nearly 0 MeV. As discussed supra, the position of the Bragg peak along a hadron beam path, **Xp,** traversing specific tissues depends on the initial energy, **Ek,** of the hadron beam. By selecting the initial energy, **Ek,** of a hadron beam intersecting a target spot **40s** located within a target tissue, the position of the Bragg peak can be controlled to correspond to the position of the target spot.

A hadron beam can also be used for characterizing properties of tissues. For example, images can be obtained with a hadron radiography system (HRS or, in particular a proton radiography system, PRS). The doses of hadrons delivered to a target spot for characterization purposes, however, may be considerably lower than the doses delivered during a hadron therapy session which, as discussed supra, are of the order of 1 to 10 Gy. The doses of delivered hadrons of HRS for characterization purposes are typically of the order of 10⁻³ to 10⁻¹ Gy (i.e. one to four orders of magnitude lower than doses typically delivered for therapeutic treatments). These doses have no significant therapeutic effects on a target spot. Alternatively, a treatment hadron beam delivered to a small set of target spots in a target tissue may be used for characterization purposes. The total dose delivered for characterization purposes is not sufficient to treat a target tissue.

As illustrated in Figure 3, downstream of the hadron source, a hadron beam of initial energy, **Ek,** is directed to the beam delivery system **12** through a beam transport line **11.** The beam transport line may comprise one or more vacuum ducts, **11v,** a plurality of magnets for controlling the direction of the hadron beam and/or for focusing the hadron beam. The beam transport line may also be adapted for distributing and/or selectively directing the hadron beam from a single hadron source **10** to a plurality of beam delivery systems for treating several patients in parallel.

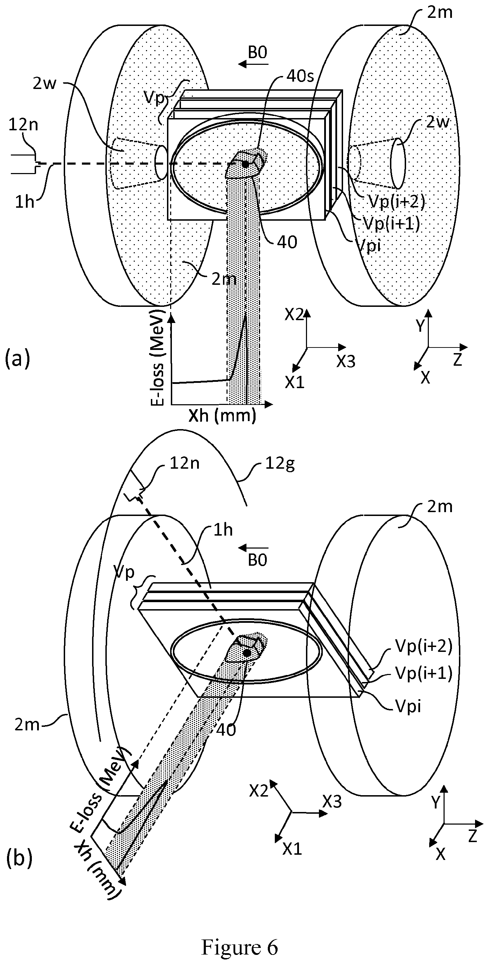

The beam delivery system **12** comprises a nozzle **12n** for orienting a hadron beam **1h** along a beam path, **Xp.** The nozzle can either be fixed or mobile. Mobile nozzles are generally mounted on a gantry **12g** as illustrated schematically in Figures 4&6. A gantry is used for varying the orientation of the hadron outlet about a circle centred on an isocentre and normal to an axis, **Z,** which is generally horizontal. In supine hadron treatment devices, the horizontal axis, **Z,** may be selected parallel to a patient lying on a couch (i.e. the head and feet of the patient are aligned along the horizontal axis, **Z).** The nozzle **12n** and the isocentre define a path axis, **Xn,** which angular orientation depends on the angular position of the nozzle in the gantry. By means of magnets positioned adjacent to the nozzle, the beam path, **Xp,** of a hadron beam **1h** can be deviated with respect to the path axis, **Xn,** within a cone centred on the path axis and having the nozzle as apex (cf. Figure 4(a)). This is advantageous in that a volume of target tissue centred on the isocentre can be treated by hadron beam without changing the position of the nozzle within the gantry. The same applies to fixed nozzles with the difference that the angular position of the path axis is fixed.

A target tissue to be treated by a hadron beam in a device provided with a gantry must be positioned near the isocentre. To this purpose, the couch or any other support for the patient can be moved; it can typically be translated over a horizontal plane **(X, Z)** wherein **X** is a horizontal axis normal to the horizontal axis, **Z,** and translated over a vertical axis, **Y,** normal to **X** and **Z,** and can also be rotated about any of the axes **X, Y, Z,** so that a central area of the target tissue can be positioned at the isocentre.

To assist in the correct positioning of a patient with respect to the nozzle **12n** according to a treatment plan previously established, the beam delivery system may comprise imaging means. For example, a conventional X-ray radiography system can be used to image an imaging volume, **Vp,** comprising the target tissue **40.** The thus obtained images can be compared with corresponding images collected previously during the establishment of the treatment plan.

Depending on the pre-established treatment plan, a hadron treatment may comprise delivery of a hadron beam to a target tissue in various forms, including the following techniques well known in the art: pencil beam, single scattering, double scattering, and uniform scattering. The present invention may apply to all hadron therapy technique. The hadron treatment, however, is preferably applied by a pencil beam technique. Figure 4 illustrates schematically this technique of delivery. A hadron beam of initial energy, **Ek**,1, is directed to a first target spot **40s1,1,** during a pre-established delivery time. The hadron beam is then moved to a second target spot **40s1,2,** during a pre-established delivery time. The process is repeated on a sequence of target spots **40s1,j** to scan a first iso-energy treatment volume, **Vt1,** following a pre-established scanning path. A second iso-energy treatment volume, **Vt2,** is scanned spot by spot following a similar scanning path with a hadron beam of initial energy, **Ek,2.** As many iso-energy treatment volumes, **Vti,** as necessary to treat a given target tissue **40** are thus irradiated following a similar scanning path. A scanning path can include several passages over a same scanning spot **40si,j.** The iso-energy treatment volumes, **Vti,** are volumes of target tissues which can be treated with a hadron beam of initial energy, **Ek,i.** The iso-energy treatment volumes, **Vti,** are slice shaped, with a thickness corresponding approximately to the breadths of the Bragg peaks at the values of the initial energy, **Ek,i,** of the corresponding hadron beams, and with main surfaces of area only limited by the opening angle of the cone centred on the path axis, **Xn,** enclosing the beam paths, **Xp,** available for a given position of the nozzle in the gantry or in a fixed nozzle device. In case of a homogeneous target tissue, the main surfaces are substantially planar as illustrated in Figure 4(b). In reality, however, since both target tissue **40** and upstream tissues **41-43** are not homogeneous in nature and thickness, the main surfaces of an iso-energy volume, **Vti,** are bumpy. The egg-shaped volumes in Figure 4(b) schematically illustrate the volumes of target tissue receiving a therapeutic dose of hadron by exposure of one target spot **40si,j** to a beam of initial energy **Ek,i.**

The dose, **D,** delivered to a target tissue **40** is illustrated in Figure 4(c). As discussed supra, the dose delivered during a treatment session is usually of the order of one to several Grays (Gy). It depends on the doses delivered to each target spot **40si,j**, of each iso-energy treatment volume, **Vti.** The dose delivered to each target spot **40si,j** depends on the intensity, **I,** of the hadron beam and on the irradiation time **tij** on said target spot. The dose, **Dij,** delivered to a target spot **40si,j** is therefore the integral, **Dij** = ∫**I** dt, over the irradiation time **tij.** A typical dose, **Dij,** delivered to a target spot **40si,j** is of the order of 0.1-20 cGy. The dose, **Di,** delivered to an iso-energy treatment volume, **Vti,** is the sum over the n target spots scanned in said iso-energy treatment volume of the doses, **Dij,** delivered to each target spot, **Di** = ∑ **Dij,** for **j** = 1 to n. The total dose, **D,** delivered to a target tissue **40** is thus the sum over the **p** irradiated iso-energy treatment volumes, **Vti,** of the doses, **Di,** delivered to each energy treatment volume, **D** = ∑ Di, for i = 1 to p. The dose, **D,** of hadrons delivered to a target tissue can therefore be controlled over a broad range of values by controlling one or more of the intensity, **I,** of the hadron beam, the total irradiation time **tij** of each target spot **40si,j,** and the number of irradiated target spots **40si,j.** Once a patient is positioned such that the target tissue **40** to be treated is located at the approximate position of the isocentre the duration of a hadron treatment session therefore depends mainly on the values of:
- the irradiation time, **tij,** of each target spot **40si,j,**
- the scanning time, **Δti,** for directing the hadron beam from a target spot **40si,j** to an adjacent target spot **40si(j+1)** of a same iso-energy treatment volume, **Vti,**
- the number n of target spots **40si,j** scanned in each iso-energy treatment volume, **Vti,**
- the time, **ΔtVi,** required for passing from a last target spot **40si,n** scanned in an iso-energy treatment volume, **Vti,** to a first target spot **40s(i+1),1** of the next iso-energy treatment volume, **Vt(i+1),** and
- the number of iso-energy treatment volumes, **Vti,** in which a target tissue **40** is enclosed.

The irradiation time, **tij,** of a target spot **40si,j** is of the order of 1-20 ms. The scanning time, **Δti,** between successive target spots in a same iso-energy treatment volume is generally very short, of the order of 1 ms. The time, **ΔtVi,** required for passing from one iso-energy treatment volume, **Vti,** to a subsequent iso-energy treatment volume, **Vt(i+1),** is slightly longer because it requires changing the initial energy, **Ek,** of the hadron beam and is of the order of 1-2 s.

As illustrated in Figure 2(a)&(b), an accurate determination of the initial energy, **Ek,** of a hadron beam is clearly critical, because if the position of the Bragg peak thus determined does not correspond to the actual position of the target tissue **40,** substantial doses of hadrons could be delivered to healthy, sometimes vital organs and could possibly endanger the health of a patient. The position of the Bragg peak mainly depends on the initial energy, **Ek,** of the hadron beam and on the nature and thicknesses of the traversed tissues. Besides determining the exact position of the target tissue within a patient, the computation of the initial energy, **Ek,** of a hadron beam yielding a position of the Bragg peak corresponding to the precise position of the target tissue therefore also requires the preliminary characterization of the tissues traversed until reaching the target tissue **40.** This characterization is performed during a treatment plan established before (generally several days before) the actual hadron treatment. The actual hadron treatment can be divided in several sessions distributed over several weeks. A typical treatment plan may start by the acquisition of data, generally in the form of images of the subject of interest with a CT scan. The images thus acquired by a CT scan may be characterized, by performing one or more of the steps:
- identifying the nature of the tissues represented on the images as a function of the X-rays absorption power of the tissues, based on the comparison of shades of grey of each tissue with a known grey scale; for example, a tissue can be one of fat, bone, muscle, water, air;
- measuring the positions and thicknesses of each tissue along one or more hadron beam paths, **Xp,** from the skin to the target tissue;
- based on their respective nature, attributing to each identified tissue a corresponding hadron stopping power ratio (HSPR);
- calculating a tissue water equivalent path length, WEPLm, of each tissue m, with m = 40 to 44, upstream of and including the target tissue, based on their respective HSPR and thicknesses;
- adding the thus determined WEPLm of all tissues **m** to yield a WEPL40s, of a target spot **40s** located in the target tissue **40,** said WEPL40s corresponding to the distance travelled by hadron beam from the skin to the target spot **40s;**
- from the WEPL40s, calculating the initial energy **Ek** of a hadron beam required for positioning the Bragg peak of the hadron beam at the target spot **40s.**
Said process steps can be repeated for several target spots defining the target tissue.

### Magnetic resonance imaging device

A magnetic resonance imaging device **2** (MRI) implements a medical imaging technique based on the interactions of excitable atoms present in an organic tissue of a subject of interest with electromagnetic fields. When placed in a strong main magnetic field, **B0**, the spins of the nuclei of said excitable atoms precess around an axis aligned with the main magnetic field, **B0**, resulting in a net polarization at rest that is parallel to the main magnetic field, **B0.** The application of a pulse of radio frequency (RF) exciting magnetic field, **B1,** at the frequency of resonance, **fL,** called the Larmor frequency, of the excitable atoms in said main magnetic field, **B0**, excites said atoms by tipping the net polarization vector sideways (e.g., with a so-called 90° pulse, **B1-90**) or to angles greater than 90° and even reverse it at 180° (with a so-called 180° pulse, **B1-180**). When the RF electromagnetic pulse is turned off, the spins of the nuclei of the excitable atoms return progressively to an equilibrium state yielding the net polarization at rest. During relaxation, the transverse vector component of the spins produces an oscillating magnetic field inducing a signal which can be collected by antennas **2a** located in close proximity to the anatomy under examination.

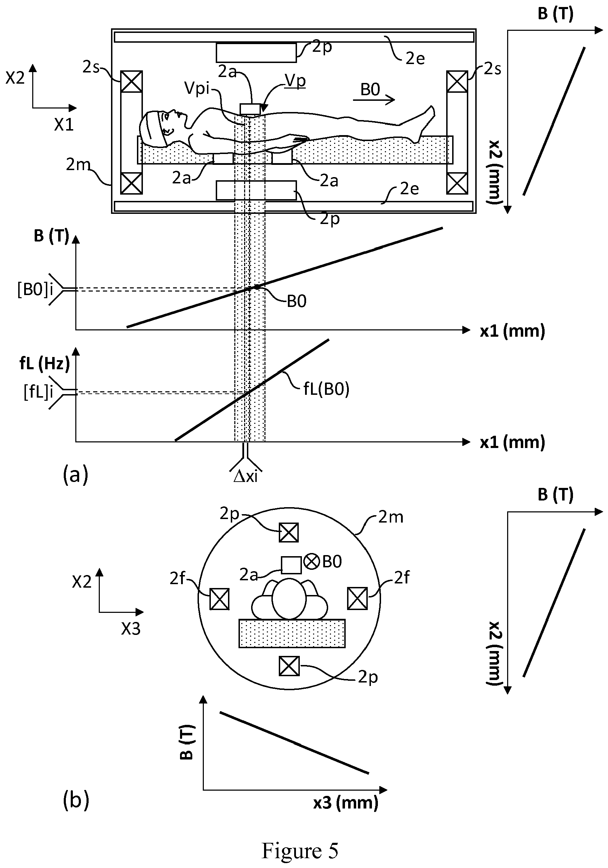

As shown in Figures 5 and 6, a MRI 2 usually comprises a main magnet unit **2m** for creating a uniform main magnetic field, **B0**; radio frequency (RF) excitation coils **2e** for creating the RF-exciting magnetic field, **B1**; X1-, X2-, and X3-gradient coils, **2s, 2p, 2f,** for creating magnetic gradients along the first, second, and third directions **X1**, **X2,** and **X3,** respectively; and antennas **2a,** for receiving RF-signals emitted by excited atoms as they relax from their excited state back to their rest state. The main magnet produces the main magnetic field, **B0**, and can be a permanent magnet or an electromagnet (a supra-conductive magnet or not). An example of a suitable MRI includes, but is not limited to, a device described in EP Pat. No. 0186238, the entire disclosure of which is incorporated herein by reference.

As illustrated in Figure 5, an imaging slice or layer, **Vpi,** of thickness, **Δxi**, normal to the first direction, **X1**, can be selected by creating a magnetic field gradient along the first direction, **X1**. In Figure 5, the first direction, **X1**, is parallel to the axis Z defined by the lying position of the patient, yielding slices normal to said axis Z. In practice, this is not necessarily the case, and the first direction, **X1**, can be any direction, e.g. transverse to the axis Z, with slices extending at an angle with respect to the patient. As shown in Figure 5(a), because the Larmor frequency, **fL,** of an excitable atom depends on the magnitude of the magnetic field it is exposed to, sending pulses of RF exciting magnetic field, **B1,** at a frequency range, **[fL]i,** excites exclusively the excitable atoms which are exposed to a magnetic field range, [**B0**]**i**, which are located in a slice or layer, **Vpi,** of thickness, **Δxi**. By varying the frequency bandwidth, **[fL]i,** of the pulses of RF exciting magnetic field, **B1,** the width, **Δxi**, and position of an imaging layer, **Vpi,** can be controlled. By repeating this operation on successive imaging layers, **Vpi,** an imaging volume, **Vp,** can be characterized and imaged.

To localize the spatial origin of the signals received by the antennas on a plane normal to the first direction, **X1**, magnetic gradients are created successively along second and third directions, **X2, X3,** wherein **X1** ⊥ **X2** ⊥ **X3,** by activating the X2-, and X3-gradient coils **2p, 2f,** as illustrated in Figure 5(b). Said gradients provoke a phase gradient, Δ**ϕ**, and a frequency gradient, Δ**f**, in the spins of the excited nuclei as they relax, which allows spatial encoding of the received signals in the second and third directions, **X2, X3.** A two-dimensional matrix is thus acquired, producing *k*-space data, and an MR image is created by performing a two-dimensional inverse Fourier transform. Other modes of acquiring and creating an MR image are known in the art and the present invention is not restricted to the selection of any particular mode.

The main magnetic field, **B0**, is generally comprised between 0.2 and 7 T, preferably between 1 and 4 T. The radiofrequency (RF) excitation coils **2e** generate a magnetic field at a frequency range, **[fL]i,** around the Larmor frequencies, **fL,** of the atoms comprised within a slice of thickness, **Δxi**, and exposed to a main magnetic field range **[B0i].** For atoms of hydrogen, the Larmor frequency per magnetic strength unit, **fL** / **B** = 42.6 MHz T⁻¹. For example, for hydrogen atoms exposed to a main magnetic field, **B0** = 2 T, the Larmor frequency, **fL** = 85.2 MHz.

The MRI can be any of a closed-bore, open-bore, or wide-bore MRI type. A typical closed-bore MRI has a magnetic strength of 1.0 T through 3.0 T with a bore diameter of the order of 60 cm. An open-bore MRI, as illustrated in Figure 6, has typically two main magnet poles **2m** separated by a gap between them for accommodating a patient in a lying position, sitting position, or any other position suitable for imaging an imaging volume, **Vp.** The magnetic field of an open-bore MRI is usually comprised between 0.2 and 1.0 T. A wide-bore MRI is a kind of closed-bore MRI having a larger diameter.

### Hadron therapy device + MRI

As discussed in the introduction with reference to Figure 2(b), the position and morphology of a target tissue **40** can evolve between a time, **t0,** of establishment of a treatment plan and a time, **t1** = **t0** + **Δt3,** of a treatment session, which can be separated by several days or weeks. A target spot **40si,j** identified in the treatment plan as belonging to the target tissue **40p** may not belong to the target tissue **40** anymore at the time, **t0** + **Δt3**, of the treatment session. The irradiation of said target spot may harm healthy tissues **43** instead of target tissues **40.**

To avoid such incidents, the prior art proposes coupling a hadron therapy device (PT) **1** to an imaging device, such as a magnetic resonance imaging device (MRI) **2.** Such coupling may not be trivial with a number of challenges to overcome but PT-MRI apparatuses have been described in the recent art and are generally known by the persons of ordinary skill in the art. For example, solutions to problems such as the correction of a hadron beam path, **Xp,** within a strong magnetic field, **B0**, of the MRI are available.

A PT-MRI apparatus allows the morphologies and positions of the target tissue and surrounding tissues to be visualized the day, **t0** + **Δt3**, of the treatment session for comparison with the corresponding morphologies and positions acquired during the establishment of a treatment plan at time, **t0.** As illustrated in the flowchart of Figure 1, in case a discrepancy of the tissues morphologies and positions between the establishment of the treatment plan at time, **t0,** and the treatment session at time, **t0** + **Δt3,** was observed, the treatment session would be interrupted and a new treatment plan would probably be established with the definition of new target spots corresponding to the actual target tissue **40** to be irradiated by hadron beams of corrected energies and directions (cf. Figure 1, diamond box "∃Δ?" → Y → "STOP"). This development of the prior art represents already a major improvement over carrying out a hadron therapy session based solely on information collected during the establishment of the treatment plan at time, **t0,** which may be obsolete at the time, **t0** + **Δt3,** of the treatment session.

The present invention aims at further improving the efficacy of a PT-MRI apparatus by providing the information required for correcting *in situ* the initial energies, Ek, and beam path, **Xp,** directions of the hadron beams, in case a change of morphology or position of the target tissue were detected. This would allow the treatment session to take place in spite of any changes detected in the target tissue **40.**

The MRI used can be any of a closed-bore, open-bore, or wide-bore MRI type described above. An open MRI affords much open space in the gap separating the two main magnet poles **2m** for orienting a hadron beam in almost any direction. Alternatively, openings or windows **2w** transparent to hadrons can be provided on the main magnet units as illustrated in Figure 6(a). This configuration has the particularity that the hadron beam can be parallel to B0. In another embodiment, a hadron beam can be oriented through the cavity of the tunnel formed by a closed bore MRI, or an annular window transparent to hadrons may extend parallel to a gantry substantially normal to the axis Z, over a wall of said tunnel, such that hadron beams may reach a target tissue with different angles. In case a fixed nozzle is used, the size of such opening or window can be reduced accordingly.

### Prompt-γ system

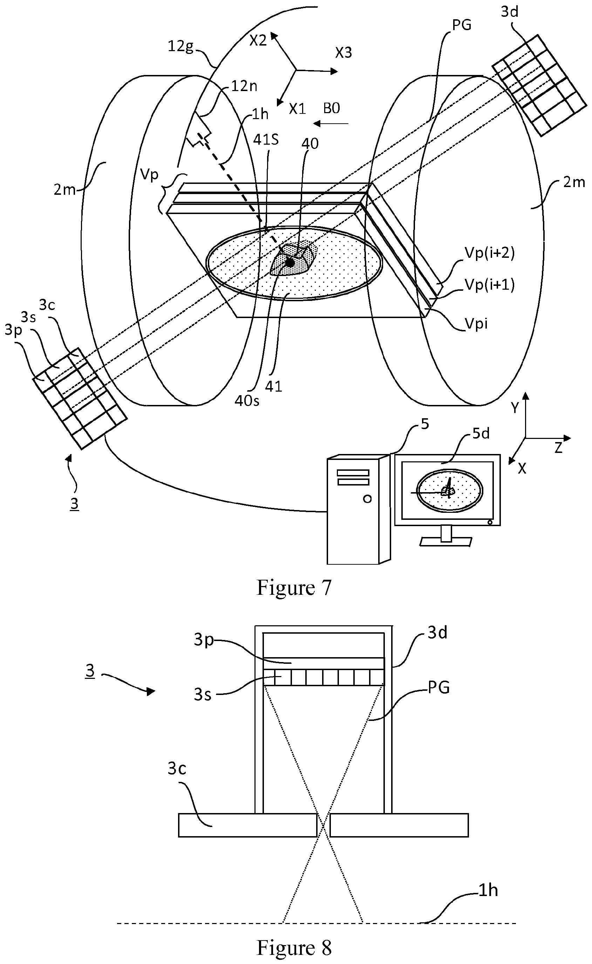

Figure 7 illustrates an example of a medical apparatus according to the present invention and comprising a hadron therapy device **1,** a magnetic resonance imaging device (MRI) **2,** and a prompt-γ (PG) system **3.**

The PG system comprises a detector **3d** configured for detecting a signal generated by a hadron beam **1h** upon interaction with the subject of interest. The hadron beam can be a treatment hadron beam having an initial beam energy **E0** comprised, for example, between 0 and 230 MeV. The hadron beam can be directed along a beam path towards a one or more target spots **40si,j** located inside the target tissue **40.** For example, the beam path of the hadron beam comes from the nozzle **12n** of the beam delivery system and goes through a subject of interest to a target spot **40s.** The beam path can also cross an outer surface **41S** of the subject of interest and one or more tissues **m** with m = 40-44.

A hadron beam that crosses material (tissues) loses a part of its energy all along its beam path. The lost is due to the interactions of the hadrons with the electrons of the tissues traversed and to the interactions with the atomic nuclei of the tissues traversed. The lost is proportional to the thickness **Lm** of the tissue **m** traversed and depends on the nature of the tissue **m.** In hadron therapy, the tissue traversed by a hadron beam are, for example, skin, fat, muscle, bone, air, water (blood), organ, and tumour. A part of the energy lost along the beam path by the hadron beam is due to inelastic interactions (i.e. collisions) between the hadrons of the hadron beam and atomic nuclei of the tissues traversed. The interactions excite the atomic nuclei bringing them in a higher energy state than the ground state before interactions. The atomic nuclei rapidly return to their ground state by emitting a prompt γ-ray. The emission of PG occurs along the beam path and its intensity depends on the probability of interaction of a hadron with an atomic nuclei and therefore on the energy of the hadron. The PG emission profile follows a curve correlated with the Bragg curve. Note that the position of the fall-off of the PG is not exactly the same as the position of the dose fall off of the Bragg curve. The PG peak usually occurs few (2-3) mm before the Bragg peak. The emission spectrum of PG is dominated by several discrete lines from specific nuclear de-excitation in the range 1-15 MeV and is isotropic. Because of their high characteristic energies, PG can escape the subject of interest with high probability and they can be detected with the PG system thus offering the possibility to retrieve the beam penetration depth (position of the Bragg peak) within the subject of interest.

The detector **3d** of the PG system can detect a signal generated by the PG emitted along the beam path. This signal allows computing the position of the Bragg peak of the hadron beam. Several techniques depending on the signal acquired, can be used to measure the position of the Bragg peak:
- PG imaging technique uses the incidence of the detected γ to determine its emission point;
- PG timing technique uses the time-of-flight of the detected γ to determine the distance from the detector to the position where the γ ray has been emitted along the hadron beam;
- PG spectroscopy technique uses the distribution of energy of the detected γ emitted at a given position along the beam path to retrieve the energy of the hadron beam at the given position.

The computation of the position of the Bragg peak within the subject of interest can be perform by simulating the PG emission of a simulated hadron beam. The simulation is then compared with the measured emission and, in case of discrepancy, is corrected.

The Figure 8 shows an example of a detector **3d** of the PG system **3** usually comprises a collimator **3c,** a scintillator **3s,** and a photon counting device **3p.** The scintillator usually comprises a scintillating material which interacts with PG to generate visible photons. The scintillator can be segmented or not, each segment corresponding to a portion of the field of view of the detector. The collimator may comprise a longitudinal slit-shaped opening **3o**. Preferably, the opening **3o** of the collimator **3c** is configured to select the PG emitted normally to the beam path. The photon counting device **3p** can also comprise a photomultiplier. As known in the art, a PG, selected by the collimator, interact with the scintillator. Then, visible photons can be multiplied with the photomultiplier to increase the signal that is acquired with the photon counting device. An example of a suitable PG detector, but is not limited to, a device described in EP Pat. application No. EP2977083A1, the entire disclosure of which is incorporated herein by reference.

The medical apparatus according to the present invention comprises:
(A) a hadron therapy device comprising a hadron source adapted for directing a hadron beam having an initial beam energy, **E0**, along a beam path to a target spot **40s** located inside a subject of interest;
(B) a MRI for acquiring magnetic resonance (MR) image within an imaging volume, Vp, comprising the target spot;
(C) a PG system adapted for acquiring a signal generated by the hadron beam;
(D)a controller configured for:
   - computing an actual position, **BP1**, of the Bragg peak of said hadron beam, based on the signal acquired by the PG system; and
   - locating the actual position, **BP1**, of the Bragg peak on an MR image of the imaging volume, **Vp,** acquired with the MRI along the beam path from an outer surface **41S** of the subject of interest to the target spot **40s.**

The water equivalent path length and thus the energy of a treatment hadron beam to a target tissue can change between the establishment of a treatment plan and a treatment session. For example, a patient having a cerebral tumour, can have a flu that fulfil the sinus of water instead of air. The water modifies the water equivalent path length computed during the treatment plan and thus the target spots located behind the sinus and along the beam path are not correctly irradiated. The apparatus of the present invention allows correcting the energy of target spots during the treatment session and thus preventing the irradiation of healthy tissues instead of target tissue. The apparatus measures the position of the Bragg peak of one or some target spots (1-20) of a same iso-energy volume (typically comprising 100 target spots) and locates the measures on a MR image. From that, a controller can calculate if modifications have been occurred between the treatment plan and the treatment session. It can then adapt the treatment session for the remaining target spots.

Preferably, the MR image provided by the MRI and the signal generated by the hadron beam provided by the PG system are acquired simultaneously or with a short delay. The two measures are thus representative of the same configuration of the tissues.

Advantageously, the plan of the MR image comprises the beam path of the (imaging) hadron beam. The MR image can be used to (help to) determine the nature of the tissues **m** traversed by the hadron beam and to determine the thicknesses **Lm** of the tissues **m** traversed by the hadron beam. It is therefore useful to image the plan in which the imaging hadron beam passes.

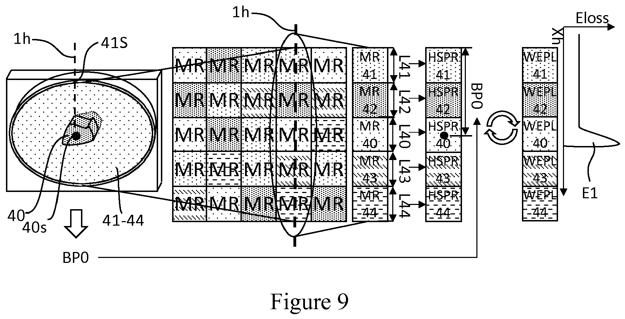

As illustrated in Figure 9, the controller 5 can acquire the signal provided by the PG system and the MR image provided by the MRI. The MR image can be used to identify the position of the outer surface **41S** of the subject of interest. The tissue traversed by the hadron beam can be selected on the MR image and the controller can determine or estimate:
- the nature of the tissues **m** traversed by the hadron beam;
- an **HSPR,m** of each tissue **m;**
- the thickness, **Lm** of the tissues **m.**

The controller can then use the signal provided by the PG system and the information from the MR image to compute the actual position, **BP1** of the Bragg Peak of the hadron beam. Usually, the computation is an iterative process. The emission of PG of a hadron beam in the traversed tissue is simulated. The simulation is compared to the measured signal. In case of difference, the simulation is adapted (for example in modifying the estimated **HSPR**,**m** of some tissues **m,** or in modifying the estimated thickness, **Lm)** and a hadron beam is again simulated. This procedure is done until the simulation and the measured signal are (nearly) the same. Practically, the computation is done until the difference between the simulation and the measured signal is smaller than a given tolerance.

Alternatively, the initial **HSPR**,**m**, **Lm,** and nature of the tissues **m** used in the simulation can be those computed during the treatment plan that are usually more accurate.

The controller may then compare the actual position, **BP1**, of the Bragg Peak with the actual position, **P1**, of the target spot **40s** targeted by the hadron beam.

The position of the Bragg peak depends on the initial energy **E0** of a hadron beam and on a water equivalent path length of the hadron beam. Knowing the position of the Bragg peak and the initial energy **E0** of the hadron beam allows computing the water equivalent path length **WEPL40s** corresponding to the water equivalent path length between the outer surface **41S** of the subject of interest and the target spot **40s.** The energy lost in the air before the outer surface of the subject of interest is usually negligible.

In case the actual position, **BP1**, and the actual position, **P1**, of the target spot **40s** are offset by a distance greater than a given tolerance, the controller can compute the water equivalent path lengths **(WEPLm)** of each tissue **m,** crossed by the beam path and comprised between the outer surface **41S** and the target spot **40s.**

The computation uses the data extracted from MR images (the nature of the tissues **m, HSPR,m,** thickness, **Lm)** and the **WEPL40s** obtained from PG system. The controller can also use the data computed during the treatment plan to improve the accuracy and the speed of the computation. For example, the controller can identify the (morphological) difference between the MR image of the treatment session and the CT (or MR) image of the treatment plan thus indicating the parameters that have to be changed in the computation.

The tolerance is preferably lower than ±10 mm, preferably ± 5 mm, more preferably ± 3 mm. In practice, a person of ordinary skill in the art knows how to estimate range uncertainties in hadron therapy applying e.g. Monte Carlo simulations. The tolerance can also be dependent on the expected precision of the detector for the target spot measured which dependents (from the most to the least important) on: the number of hadrons stopping on the target spot, the distance, the beam energy and the nature of the tissue. The tolerance thus might be dependent on the subject of interest and/or on the target spot.

The apparatus according to the invention preferably further comprises a display **5d** and the controller is configured for representing on a same coordinate scale the MR image obtained from the MRI and the position of the Bragg peak obtained from the PG system.

The **WEPLm** can be used to correct the planned initial beam energy **E0** of target spot **40s.** The energy **E0** is increased or decreased to a corrected initial energy **E1** such that the position of the Bragg peak of the hadron beam correspond to the position of the target spot **40s.** The corrected initial beam energy **E1** ≡ **E1,i** is then suitable for matching the positions of the Bragg peak of said hadron beam with the positions of all the other target spots **40si,j** located in a same iso-energy volume, **Vti.**

Preferably, the **WEPLm** are computed for several spots of the same iso-energy volume, **Vti** in order to increase the reliability of the computation and to avoid local effects (such as the above example of the water in the sinus).

The computation of the corrected initial beam energy **E1** may thus be performed on the basis of some target spots within a same iso-energy volume, **Vti.** As discussed with respect to Figure 4(c), this can be achieved either by irradiating few target spots, e.g., irradiating 1 to 40% of the target spots of an iso-energy layer, **Vti,** preferably 5 to 30%, more preferably 10 to 20%. The dose delivered by the hadron beam directed towards these spots is thus not sufficient to treat the target tissue because in case of a change of morphology of the tissues, a full therapeutic dose reaching healthy tissues would be extremely detrimental to the health of a patient. In these conditions, the validation of the treatment plan is safe for the patient, even if a correction of the initial energy is required. In practice, the corrected initial energy, **E1**, can be used during the treatment session to treat all the target spots **40si,j** of an iso-energy volume, **Vti.** The initial energies required for treating target spots, **40(i+1),j,** ... in subsequent iso-energy volumes, **Vt(i+1),** ..., can either be extrapolated from the initial energy, E1, determined for the iso-energy volume, **Vti,** or, alternatively or additionally, a selection of target spots **40(i+1),j,** ... of the subsequent energy volumes, **Vt(i+1),** ... can be tested as described above

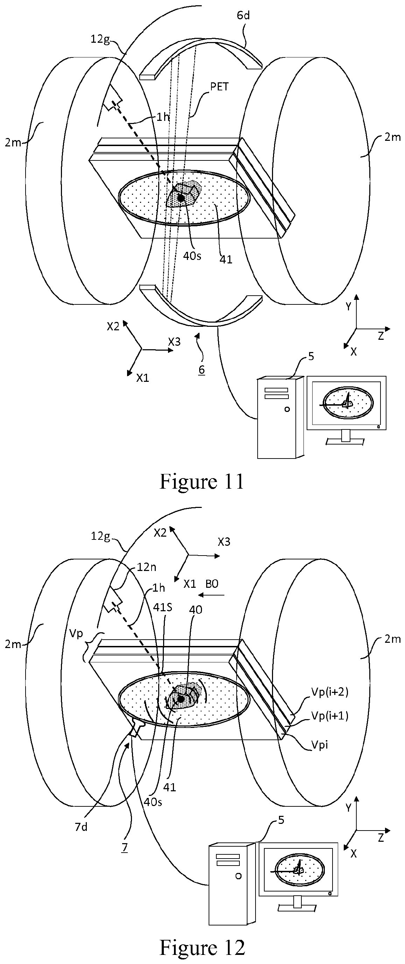

Alternatively, as illustrated in Figure 11, the PG system may be replaced by a positron emission tomography (PET) scan **6** can be used. A PET scan is a device for imaging in 3D the concentration of β⁺ (positron) emitter located along the hadron beam path in the subject of interest. A small fraction of the hadrons of the hadron beam create positron emitting isotopes (for example, 11C, 13N, 150) through interactions with the atomic nuclei of the tissues traversed. These radio-active isotopes decay with emission of a positron which will annihilate with an electron leading to the emission of two gamma photons emitted in coincidence. The detector **6d** of the PET scan detects the source of emission of these two gamma photons and therefore measures the concentration of β⁺ emitter. The concentration of β⁺ emitter is related to the beam path of the hadron beam.

In yet another alternative, an ultrasound system can replace the PG system. The Figure 12 shows an example of ultrasonic system 7 comprising an ultrasonic detector **7d.** An example of a suitable ultrasonic system, but is not limited to, a device described in Assmann, W., Kellnberger, S., Reinhardt, S., Lehrack, S., Edlich, A., Thirolf, P. G., Parodi, K. (2015). Ionoacoustic characterization of the proton Bragg peak with submillimeter accuracy. Medical Physics, 42(2), 567-74. http://doi.org/10.1118/1.4905047, the entire disclosure of which is incorporated herein by reference.

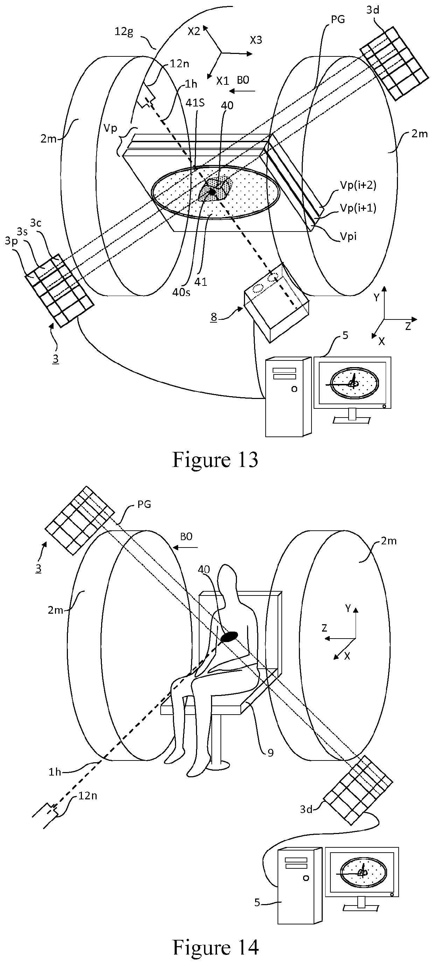

The medical apparatus according to the present invention can also comprise a hadron radiography system (HRS, or in particular a proton radiography system, PRS) **8** as shown on Figure 13. A HRS uses an imaging hadron beam that crosses the subject of interest (and the target spot) and can measure the water equivalent path length, **WEPL,HRS,** of the subject of interest crossed by the hadron beam. This **WEPL,HRS** provide additional information on the position of the Bragg peak and can be used to improve the **WEPLm** determination. It thus allows an improvement of the range determination of the hadron beam. The detector of the HRS can be one of the following detector: a range telescope, a calorimeter or a spectrometer.

Figure 14 illustrates an example of the hadron therapy device according to the present invention further comprising a support **9** for supporting a patient in a non-supine position. A low uncertainty on the actual position of the target tissue **40s** can permit morphological differences between the establishment of the treatment plan and the treatment session. In this context, treating a patient in a non-supine position can be advantageous because it does not require a gantry. The beam nozzle is thus fixed and the cost of the apparatus is greatly decreased. The apparatus according to the embodiment of the Figure 14 can also comprise a HRS.

According to a second aspect, the present invention relates to a method for locating the Bragg peak of a hadron beam having an initial beam energy, **E0** and being emitted along a beam path to a target spot **40s** within a target tissue **40.** The location of the Bragg peak of a hadron beam with respect to the target spot allows verifying a treatment plan previously established.

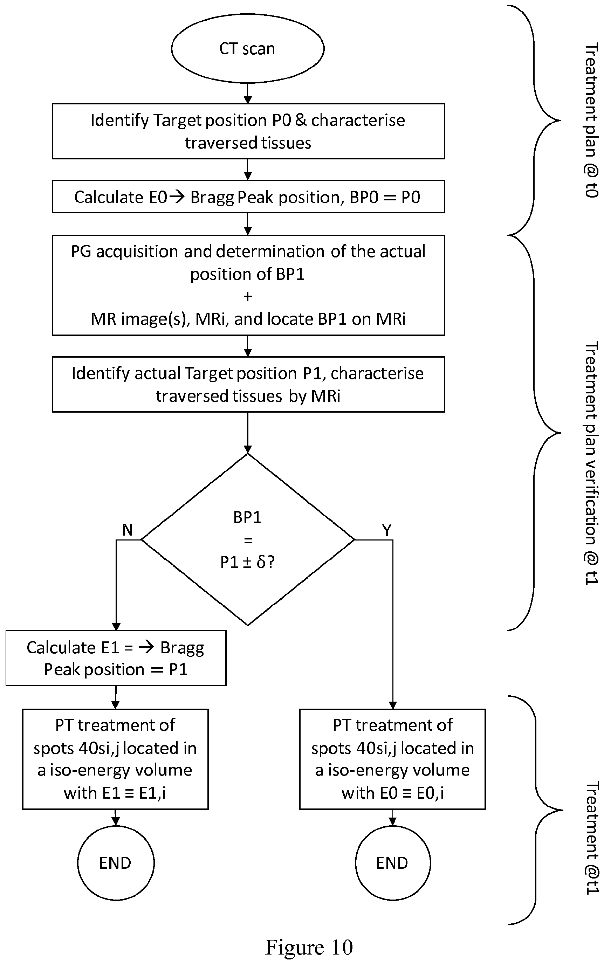

Figure 10 illustrates an example of flowchart according to the method of the present invention. Firstly, a classical treatment plan can be established at a time **t0,** using a CT scan such as described above. A typical treatment plan may provide images of the subject of interest with a CT scan. The images permit to identify the position, **P0**, of a target spot of the target tissue **40** and to characterize the tissues traversed by the hadron beam. A treatment plan system then computes the initial beam energy, **E0**, such that the position, **BP0,** of the Bragg peak corresponds to the position, **P0**, of the target sport of the target tissue. These operations may be repeated for several target spots **40si,j.**

The method according to the present invention is performed, for example, during a treatment session. As illustrated in Figure 10, the localisation of the Bragg peak of a hadron beam is the first step of the method. A magnetic resonance (MR) imaging of an imaging volume, **Vp,** comprising a target spot **40s** is performed and MR image is acquired. Then a PG system can detect and acquire a signal generated by a hadron beam having an initial beam energy, **E0** and being emitted, along the beam path to the target spot **40s.**

As described above, the signal acquired by the PG system allows computing the actual position, **BP1**, of the Bragg peak of the hadron beam. The actual position **BP1** can then be located on the MR image.

The method of the present invention can also comprise a comparison of the actual position **BP1** of the Bragg peak with the actual position, **P1**, of the target spot **40s** determined from the MR image during the verification of the treatment plan, for example, during a treatment session at the time **t1** later than **t0.**

In the case the actual position, **BP1**, of the Bragg peak, and the actual position, **P1**, of the target spot **40s** are offset by a distance greater than a given tolerance **δ**, a correction of the initial energy of the hadron beam may be performed. To achieve this correction, the water equivalent path lengths **WEPLm** of each tissue **m** crossed by the beam path and comprised between the outer surface **41S** and the target spot **40s** are computed. The computation is based on the thickness **Lm** and nature of each tissue **m** determined on the MR image. The computation also uses the water equivalent path length **WEPL40s** corresponding to the distance between the outer surface **41S** and the target spot **40s.** The **WEPL40s** can be determined by the PG system using the knowing of the actual position **BP0** of the Bragg peak.

The tolerance **δ** on the offset between the actual position, **BP1**, of the Bragg peak, and the actual position, **P1**, of the target spot **40s** is preferably lower than ± 10 mm, preferably ± 5 mm, more preferably ± 3 mm.

The planned initial beam energy **E0** of target spot **40s** is then corrected to a corrected initial beam energy **E1**, suitable for matching the positions of the Bragg peak of said hadron beam with the actual position of target spot **40s.** This energy may also be suitable for all target spots **40si,j** located in a same iso-energy volume, **Vti,** then the target spot **40s.**

Preferably, the magnetic resonance image obtained from the MRI and the actual position, **BP1**, of the Bragg peak obtained from the PG system are represented on a display **5d** on a same coordinate scale.

As described above, the method and apparatus according to the present invention can be used to compute the actual position, **BP1,** of the Bragg peak of the hadron beam from the signal generated by the emission of PG of one or several target spots **40si,j** and acquired by the PG system. The computation is performed during a treatment session. The total treatment usually comprises several treatment sessions and the time between the first and the treatment session may be separated by several days or weeks. Preferably, the computation is thus performed during several treatment sessions at time **t0** + **Δt1, t0** + **Δt2, t1** = **t0** + **Δt3** for, at least, a part of the target spots **40si,j.**

The measure acquired during the treatment sessions at time **t0** + **Δt1, t0** + **Δt2, t1** = **t0** + **Δt3** allow computing an evolution of the actual position, **BP1,** of the Bragg peak of one or more target spots **40si,j.** The evolution permits to observe general trends of modifications of the morphology and/or position of the target tissue (or surrounding tissue). When such trends are observed and exceed predetermined limits a new treatment plan may be established. The trends can also be used to extrapolate a treatment that will be delivered later.

Preferably, the magnetic resonance (MR) imaging and the emission of a hadron beam are done in the same room.

Preferably, the method according to the present invention is performed with a medical apparatus according to the present invention.

The aim of the present invention is to reduce the range uncertainty of a hadron beam. The use of the PG system allows measuring the position of the Bragg peak of a hadron beam within a subject of interest. The MRI then provides images that help to identify the nature and thickness of the tissues traversed by the hadron beam and to identify the outer surface of the subject of interest, thus allowing to represent on same scale the signal from the PG system and the MR image. This information may be used to check a treatment plan during a treatment session thus reducing the risk of incorrect treatment and improving the quality (adaptation of the energy) and precision (lower uncertainty) of the treatment.

## Claims

1. A medical apparatus comprising:
(A) a hadron therapy device (1) comprising a hadron source (10) adapted for directing a hadron beam (1h) having an initial beam energy, E0, along a beam path (Xp) to a target spot (40s) located inside a subject of interest;
(B) a magnetic resonance imaging device (MRI) for acquiring magnetic resonance (MR) image within an imaging volume, Vp, comprising the target spot;
**characterised in that,** the medical apparatus further comprises:
(C) a prompt-γ system (3) adapted for acquiring a signal generated by the hadron beam;
(D) a controller (5) configured for:
• computing an actual position, BP1, of the Bragg peak of said hadron beam, based on the signal acquired by the prompt-γ system; and
• locating the actual position, BP1, of the Bragg peak on an MR image of the imaging volume, Vp, acquired with the MRI along the beam path from an outer surface (41S) of the subject of interest to the target spot.

2. The medical apparatus according to claim 1, further comprising:
(E) a display (5d), and
wherein the controller is configured for representing on a same coordinate scale the MR image obtained from the MRI and the position of the Bragg peak obtained from the prompt-γ system.

3. The medical apparatus according to claim 1 or 2, wherein the controller is configured for comparing the actual position, BP1, of the Bragg peak and the actual position, P1, of the target spot (40s).

4. The medical apparatus according to claim 3,
wherein in case the actual position, BP1, and the position, P1, of the target spot (40s) are offset by a distance greater than a given tolerance, the controller is further configured for computing the water equivalent path lengths (WEPLm) of each tissue m, crossed by the beam path and comprised between the outer surface (41 S) and the target spot (40s),
said computation being based on the thickness Lm and nature of each tissue m determined on the MR image, and on the water equivalent path length (WEPL40s) corresponding to the distance between the outer surface and the target spot (40s) determined by the prompt-γ system, and
wherein said tolerance is preferably lower than ±10 mm, preferably ± 5 mm, more preferably ± 3 mm.

5. The medical apparatus according to claim 4, wherein the controller (5) is configured for optimising the treatment plan by correcting the value of planned initial beam energy E0 of target spot (40s), to a corrected initial beam energy E1, suitable for matching the positions of the Bragg peak of said hadron beam with the positions of all target spots (40si,j) located in a same iso-energy volume, Vti.

6. The medical apparatus according to anyone of the previous claims, wherein the prompt-γ system is replaced by at least one of a PET system and an ultrasound system.

7. The medical apparatus according to any one of the previous claims, further comprising a hadron radiography system.

8. The medical apparatus according to any one of the previous claims, comprising a support for supporting a patient in a non-supine position.

9. A method for locating the Bragg peak of a hadron beam having an initial beam energy, E0 and being emitted along a beam path to a target spot (40s) within a target tissue (40), said method comprising the following steps:
(A)performing a magnetic resonance (MR) imaging of an imaging volume, Vp, comprising a target spot, and acquiring MR image;
(B) emitting, along the beam path to the target spot, the hadron beam having an initial beam energy, E0;
**characterized in that** said method comprises the following additional steps:
(C) detecting a signal generated by said hadron beam with a prompt-γ system;
(D) from said signal, determining an actual position, BP1, of the Bragg peak of said hadron beam, based on the signal acquired by the prompt-γ system;
(E) locating the actual position, BP1, of the Bragg peak on an MR image of the imaging volume, Vp, acquired with the MRI along the beam path from an outer surface (41S) of the subject of interest to the target spot (40s).

10. The method according to claim 9, further comprising the step of comparing the actual position, BP1, of the Bragg peak and the actual position, P1, of the target spot (40s).

11. The method according to claim 10, wherein in case the actual position, BP1, and the actual position, P1, of the target spot (40s) are offset by a distance greater than a given tolerance, the method further comprising the step of computing the water equivalent path lengths (WEPLm) of each tissue m, crossed by the beam path and comprised between the outer surface (41 S) and the target spot, said computation being based on the thickness Lm and nature of each tissue m determined on the MR image, and on the water equivalent path length (WEPL40s) corresponding to the distance between the outer surface and the target spot determined by the prompt-γ system, and wherein said tolerance is preferably lower than ±10 mm, preferably ± 5 mm, more preferably ± 3 mm.

12. The method according to claim 11, further comprising the step of optimising the treatment plan by correcting the value of planned initial beam energy E0 of target spot (40s), to a corrected initial beam energy E1, suitable for matching the positions of the Bragg peak of said hadron beam with the positions of all target spots (40si,j) located in a same iso-energy volume, Vti.

13. The method according to any one of the claims 9 to 12, further comprising the following steps:
(F) providing a display (5d);
(G) representing on a same coordinate scale the magnetic resonance data obtained from the MRI and the actual position of the Bragg peak obtained from the prompt-γ system.

14. The method according to any one of the claims 9 to 13, wherein the steps of performing a magnetic resonance (MR) imaging and of emitting an imaging hadron beam are done in the same room.

15. A method according to anyone of the claims 9 to 14, further comprising the step of providing a medical apparatus according to anyone of the claims 1 to 8.
